# EUROPÄISCHE PATENTANMELDUNG

(11) **EP 3 748 643 A1**
(43) Veröffentlichungstag der Anmeldung: **09.12.2020**
(21) Anmeldenummer: 19178385.1
(22) Anmeldetag: 05.06.2019
(51) Int. Cl.: G16H 40/20

(54) **STEUERUNG DER ÜBERTRAGUNG VON MEDIZINISCHEN BILDDATENPAKETEN ÜBER EIN NETZWERK**

(71) Anmelder: Siemens Healthcare GmbH, 91052 Erlangen (DE)
(72) Erfinder: Igler, Harald, 91353 Hausen (DE)

(57) **Zusammenfassung**

Die vorliegende Erfindung betrifft die Steuerung der Übertragung von medizinischen Datenpakten (p) über ein Netzwerk (NW). Unter Zugriff auf ein trainiertes Modell (M) wird ein optimaler Zeitpunkt für die Datenübertragung unter Berücksichtigung der jeweiligen Überragungserfordernisse, die das Datenpaket (p) hat und unter Berücksichtigung der aktuellen Netzwerk-Kenndaten (kd)ermittelt. Das Datenpaket (p) kann, falls erforderlich, dazu in einem Pufferspeicher (PS) zwischengespeichert werden.

## Beschreibung

Die vorliegende Erfindung betrifft die Steuerung der Übertragung von Datenpakten, wie z.B. hoch-volumigen medizinischen Bilddaten, über ein Netzwerk von einem Sendeknoten an einen Empfangsknoten.

Insbesondere im medizinischen Bereich steigt die Menge an zu übertragenden Daten kontinuierlich. Dies ist nicht zuletzt darin begründet, dass mehr und mehr automatische Analysemethoden zur Auswertung der Daten existieren und angewendet werden. So stehen z.B. immer mehr Kliniken in Datenaustausch, um eine bessere medizinische Versorgung sicherstellen zu können (indem z.B. Experten, die an entfernten Rechnern arbeiten, eingebunden werden können oder indem erfasste Bilddaten an externen Rechenzentren nachbearbeitet werden). Darüber hinaus soll die Möglichkeit gegeben werden, ein System fernzusteuern und z.B. mit Hilfe von Kamerasystemen interventionelle Operationen durchzuführen. Dies erfordert einen stabilen, sicheren Datenfluss.

Dazu ist somit ein stabiles Netzwerk zur sicheren Datenübertragung unabdingbar.

Gerade die Übertragung von Datenpakten mit einem hohen Datenvolumen kann das Netzwerk belasten, was möglicherweise zu Überlastungen des Netzwerkes und sogar mitunter zu Störungen führen kann.

Da die Netzwerke in den unterschiedlichen Ländern und Regionen eines Landes nicht gleichermaßen ausgebaut sind, kann es aufgrund der technischen Netzwerkbeschränkungen in der Praxis zu Störungen kommen. Damit kann eine sichere Übertragung der Daten nicht immer gewährleistet werden.

Der vorliegenden Erfindung liegt die Aufgabe zugrunde, technische Mittel bereitzustellen, um auch dann eine zuverlässige und sichere Übertragung von Datenpaketen sicher stellen zu können, wenn das zugrundeliegende Netzwerk zumindest phasenweise hinsichtlich seiner Übertragungskapazitäten beschränkt ist.

Diese Aufgabe wird jeweils durch die Gegenstände nach den unabhängigen, einander nebengeordneten Ansprüchen, insbesondere durch ein Verfahren, einen Steuerknoten, ein Computerprogramm und ein Computerprogrammprodukt gelöst. Vorteilhafte Ausführungsformen sind Gegenstand der abhängigen Ansprüche, der Beschreibung und der Zeichnungen.

Gemäß einem ersten Aspekt wird die Aufgabe durch ein Computer-implementiertes Verfahren zum Erzeugen eines Sendebefehlssatzes zur Steuerung der Übertragung (insbesondere der Versendung) von Datenpaketen über ein digitales Netzwerk gelöst, bei dem ein trainiertes Modells (wie z.B. ein trainiertes, neuronales Netz) bereitgestellt ist, das in einem Speicher gespeichert ist und das trainiert ist, um für ein jeweiliges Datenpaket mit entsprechenden Übertragungserfordernissen unter Berücksichtigung von Netzwerk-Kenndaten einen Sendebefehlssatz derart zu berechnen, dass die Übertragungserfordernisse bei Übertragung des Datenpaktes erfüllt werden können. Dazu umfasst das Verfahren folgende Verfahrensschritte:
- Erfassen des zu übertragenden Datenpaketes;
- Berechnen von Übertragungserfordernissen des zu übertragenden Datenpaketes;
- Erfassen von aktuellen Netzwerk-Kenndaten;
- Anwenden des trainierten Modells mit den erfassten Übertragungserfordernissen und den erfassten aktuellen Netzwerk-Kenndaten zur Berechnung und Bereitstellung des Sendebefehlssatzes.

Im Kern betrifft die Anmeldung eine intelligente Steuerung der Übertragung der Datenpakete, indem die Bedingungen für die Übertragung (wann soll übertragen werden, in welcher Form: das komplette Datenpaket oder in Form von zerlegten Subpakten, verschlüsselt, nach welchen Übertragungsprotokoll etc.) dynamisch und in Abhängigkeit von den aktuell erfassten Netzwerkbedingungen (wie z.B. der Netzwerkauslastung) und unter Zugriff auf ein Modell, in dem Wissen über die Übertragung von Datenpaketen gespeichert ist, berechnet werden. Bei dem Modell kann es sich z.B. um ein trainiertes neuronales Netzwerk handeln.

In einer vorteilhaften Ausführungsform der Erfindung dient das trainierte Modell zur Anwendung von Verfahren zum maschinellen Lernen. Dabei können insbesondere die real gemessenen Übertragungslaufzeiten dem trainierten Modell in einer Rücckopplungsschleife zurückgespeist werden, um somit eine Nachregelung des Modells bereitzustellen und das System selbstlernend auszubilden.

In einer vorteilhaften Ausführungsform umfasst der Sendebefehlssatz einen Zeitparameter und/oder einen Zerlegungsparameter, wobei der Zeitparameter definiert, zu welchem Zeitpunkt der Sendebefehl umgesetzt werden soll und wobei der Zerlegungsparameter definiert, ob und wenn ja, wie das zu übertragende Datenpaket in Teilpakete zerlegt werden soll, die unabhängig und getrennt voneinander übertragen werden. Damit können der Übertragungszeitpunkt und/oder die Art und Weise der Übertragung des Datenpaketes an die aktuellen Netzwerkbedingungen optimiert angepasst werden. Mit dieser Maßnahme kann sichergestellt werden, dass die Datenübertragung auch dann zuverlässig erfolgt, wenn das Netzwerk teilweise überlastet ist oder keine ausreichende Performance bereitstellen kann. Dies ist insbesondere in Ländern hilfreich, die nicht über ein ausreichend ausgebautes Netzwerk verfügen.

In einer vorteilhaften Ausführungsform ist das trainierte Modell mit Trainingsdaten und einem überwachten Lernverfahren (supervised learning) trainiert worden. Beim ,supervised learning' wird ein konkretes Ergebnis (Sendebefehlssatz) für die unterschiedlichen Eingabemöglichkeiten (Übertragungserfordernisse und Netzwerk-Kenndaten) vorgegeben. Anhand des ständigen Vergleichs zwischen Soll- und Ist-Ergebnis lernt das Netz die Neuronen passend zu verknüpfen. Die Trainingsdaten können Plandaten (z.B. Zeitparameter, wie Laufzeit, Rückmeldungen zur Laufzeit, Geschwindigkeitsparameter oder Anzahl fehlerhafter Paket/daten), Referenzdaten von vergleichbaren System und/oder Simulationsdaten und/oder historische Daten umfassen. Zu den Simulationsdaten sei folgendes bemerkt: Ein klinisches Netzwerk besteht aus unterschiedlichsten Komponenten (z.B. diverse Scanner (MR- und/oder CT-, Ultraschall-Gerät etc. Patientendaten- und Abrechnungssystem). Die Performance innerhalb des Kliniknetzes und Abhängigkeit (Störungen -Fehleranfälligkeit) kann vorab simuliert werden .Dabei sind teilweise bis zu 150 Netzwerknoten (teils Dicom-Knoten) zu beachten oder fließen in die Simulation mit ein. Diese Simulationsdaten werden berücksichtigt.

Verfahren nach einem der vorangehenden Patentansprüche, bei dem die zu übertragenden Datenpakete nach Priorität kategorisiert werden, wobei die Priorität vorzugsweise vom Sendeknoten automatisch und insbesondere durch Anwendung eines Analysealgorithmus auf den Inhalt des Datenpaketes bestimmt wird.

In einer vorteilhaften Ausführungsform werden die zu übertragenden Datenpakete mit einem Kennungsfeld erweitert, wobei das Kennungsfeld zumindest eine Angabe zur Priorität des Datenpaktes umfasst. Die Priorität kann vom Sendeknoten definiert werden. Die Priorität kann durch einen Priorisierungsalgorithmus automatisch berechnet werden. Dabei kann z.B. berücksichtigt werden, ob es sich um eine Routinesendung handelt, um Ausfalldaten oder um ein Datenpaket, das einen Totalausfall des Systems indiziert. Damit kann die verfügbare Netzwerkkapazität besser ausgenützt werden.

In einer weiteren, vorteilhaften Ausführungsform werden die zu übertragenden Datenpakete mit einem Anforderungsfeld erweitert, wobei das Anforderungsfeld eine Aktivierungsfunktion umfasst, über die das jeweilige Datenpaket von einem externen Empfangsknoten angefordert werden kann. Damit kann sozusagen ein PULL-Betrieb umgesetzt werden, auch wenn der Normalfall ein PUSH-Betrieb realisiert ist, bei dem die Übertragung von Sendeknoten aus getriggert wird, während beim PULL-Betrieb die Übertragung vom Empfangsknoten getriggert ist.

Vorstehend wurde die Lösung der Aufgabe anhand des Verfahrens beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Ansprüche (die beispielsweise auf einen Steuerknoten oder auf ein Computerprogrammprodukt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

In einem weiteren Aspekt betrifft die Erfindung einen Steuerknoten zur Steuerung der Übertragung (insbesondere zur Versendung) von Datenpaketen von einem Sendeknoten an einen Empfangsknoten über ein digitales Netzwerk. Der Steuerknoten ist ausgebildet, ein Verfahren, wie vorstehend beschrieben, auszuführen. Dabei kann der Steuerknoten weitergebildet sein, um die oben beschriebenen alternativen Ausführungsformen und/oder Merkmale des Verfahrens zu implementieren.

In einer weiteren bevorzugten Ausführungsform der Erfindung umfasst der Steuerknoten eine erste Schnittstelle zur Erfassung des Datenpaktes mit den Übertragungserfordernissen und/oder eine zweite Schnittstelle zur Erfassung von aktuellen Netzwerk-Kenndaten und/oder eine dritte Schnittstelle zur Ausgabe des Sendebefehlssatzes und/oder zum Empfang von real gemessenen Messdaten zu der ausgeführten Datenübertragung. In alternativen Ausführungsformen der Erfindung kann der Empfang von Daten noch auf weitere Schnittstellen aufgeteilt werden, so dass beispielsweise der Empfang des Datenpaktes über eine andere Schnittstelle empfangen wird als die Übertragungserfordernisse, wenn diese nicht direkt in dem Steuerknoten berechnet werden, was ebenfalls möglich ist. Dazu wird auf dem Steuerknoten eine Funktionalität (z.B. in Form eines Algorithmus) bereitgestellt, mit der aus dem Datenpaket unter Zugriff auf z.B. historische Daten und/oder unter Zugriff auf das Modell die Übertragungserfordernisse automatisch berechnet werden.

In einer weiteren bevorzugten Ausführungsform der Erfindung umfasst der Steuerknoten einen Modellspeicher. Alternativ kann der Modellspeicher auf einem externen Knoten angeordnet sein, auf den der Steuerknoten zugreifen kann, in dem das trainierte Modell abgelegt ist.

In einer weiteren bevorzugten Ausführungsform umfasst der Steuerknoten einen Speicher oder kann auf einen Speicher zugreifen, in dem das zu übertragenden Datenpaket vor Versendung zwischengespeichert ist. Damit ist sichergestellt, dass alle zu übertragenden Datenpakete sicher übertragen werden und dabei die Übertragung in Hinblick auf die aktuellen Netzwerkbedingungen angepasst und optimiert ist.

In einem weiteren Aspekt betrifft die Erfindung ein Computerprogrammprodukt mit Programmcode für das Ausführen eines Verfahrens wie oben beschrieben, wenn das Computerprogramm auf einem elektronischen Gerät oder einem Computer ausgeführt wird.

In einem weiteren Aspekt betrifft die Erfindung ein Computerprogramm mit Programmcode für das Ausführen eines Verfahrens wie oben beschrieben, wenn das Computerprogramm auf einem elektronischen Gerät oder einem Computer ausgeführt wird. Dabei ist es auch möglich, dass das Computerprogramm auf einem von einem Computer lesbaren Medium, wie z.B. einem internen oder externen Speicher, gespeichert ist. Das Computerprogramm kann in verteilter Weise ausgeführt werden, so dass beispielsweise vereinzelte Verfahrensschritte auf einer ersten Recheneinheit und andere Verfahrensschritte auf einer zweiten Recheneinheit ausgeführt werden. Zudem können vereinzelte Verfahrensschritte in einer anderen Reihenfolge ausgeführt werden. Letzteres betrifft insbesondere das Erfassen des Datenpaketes, das Berechnen von Übertragungserfordernissen und das Erfassen des aktuellen Netzwerkstatus mit den Kenndaten. Ferner können vereinzelte Verfahrensschritte wiederholt ausgeführt werden.

Ein wichtiger Aspekt der vorliegenden Anmeldung liegt in der adaptiven Anpassung (Nachregelung) des Modells. Damit wird das System selbstlernend und kann aus den fortlaufend gesammelten Daten und insbesondere unter Berücksichtigung der real gemessenen Übertragungsdaten (der Messdaten) das Modell schrittweise verfeinern und justieren. Des Weiteren können dem Modell auch externe Daten, z.B. historische Daten von vergangenen Übertragungen oder Daten von Referenzsystemen Eingang finden. Entsprechend können in dem Model Algorithmen zur Analyse von einer großen Menge von Daten (Big Data Analyse) angewendet werden.

Das Verfahren ist computer-implementiert und wird auf einer Recheneinheit mit einem Prozessor ausgeführt. Der Steuerknoten kann als eine solche Recheneinheit ausgebildet sein oder eine solche umfassen.

Die Recheneinheit kann in einer Maschine, z.B. in einem Computer, Personal-Computer oder als eine Workstation und/oder in virtualisierter Form in einem Computernetzwerk ausgebildet sein und umfasst einen Prozessor (Verarbeitungseinheit) und kann einen Systemspeicher und einen Systembus umfassen, der verschiedene Systemkomponenten einschließlich des Systemspeichers mit der Verarbeitungseinheit koppelt. Unter einem Prozessor soll in diesem Zusammenhang beispielsweise eine elektronische Schaltung verstanden werden. Bei einem Prozessor kann es sich insbesondere um einen Hauptprozessor (engl. Central Processing Unit, CPU), einen Mikroprozessor oder einen Mikrocontroller, beispielsweise eine anwendungsspezifische integrierte Schaltung oder einen digitalen Signalprozessor, möglicherweise in Kombination mit einer Speichereinheit zum Speichern von Programmbefehlen, etc. handeln. Bei einem Prozessor kann es sich beispielsweise auch um einen IC (integrierter Schaltkreis, engl. Integrated Circuit), insbesondere einen FPGA (engl. Field Programmable Gate Array) oder einen ASIC (anwendungsspezifische integrierte Schaltung, engl. Application Specific Integrated Circuit), oder z. B. ein Multi-Chip-Modul, z. B. ein 2,5D oder 3D Multi-Chip-Modul, bei dem insbesondere mehrere sogenannte Dies direkt oder über einen Interposer miteinander verbunden sind oder einen DSP (Digitaler Signalprozessor, engl. Digital Signal Processor) oder einen Grafikprozessor GPU (Graphic Processing Unit) handeln. Auch kann unter einem Prozessor ein virtualisierter Prozessor, eine virtuelle Maschine oder eine Soft-CPU verstanden werden. Es kann sich beispielsweise auch um einen programmierbaren Prozessor handeln, der mit Konfigurationsschritten zur Ausführung des genannten erfindungsgemäßen Verfahrens ausgerüstet wird oder mit Konfigurationsschritten derart konfiguriert ist, dass der programmierbare Prozessor die erfindungsgemäßen Merkmale des Verfahrens, der Komponente, der Module, oder anderer Aspekte und/oder Teilaspekte der Erfindung realisiert.

Die Recheneinheit kann auch ein Festplattenlaufwerk zum Lesen von und Schreiben auf eine Festplatte, ein Magnetplattenlaufwerk zum Lesen oder Schreiben auf eine (z.B. entfernbare) Magnetplatte und ein optisches Plattenlaufwerk zum Lesen oder Schreiben auf eine entfernbare (magnetische) optische Platte wie eine Kompaktplatte oder andere (magnetische) optische Medien beinhalten. Das Festplattenlaufwerk, das Magnetplattenlaufwerk und das (magnetische) optische Laufwerk können über eine Festplattenschnittstelle, eine Magnetplattenantriebsschnittstelle und eine (magnetische) optische Antriebsschnittstelle mit dem Systembus gekoppelt werden. Die Laufwerke und die zugehörigen Speichermedien bieten eine nichtflüchtige Speicherung von maschinenlesbaren Anweisungen, Datenstrukturen, Programmmodulen und anderen Daten für den Computer. Obwohl die hierin beschriebene exemplarische Umgebung eine Festplatte, eine austauschbare Magnetplatte und eine austauschbare (magnetische) optische Platte verwendet, wird der Fachmann wissen, dass andere Arten von Speichermedien, wie z.B. Flash-Speicherkarten, "Random Access Memory" (RAMs), "Read Only Memory" (ROM) und dergleichen, anstelle oder zusätzlich zu den oben vorgestellten Speichervorrichtungen verwendet werden können. Ein grundlegendes Ein-/Ausgabesystem (BIOS) kann im ROM gespeichert werden, das grundlegende Routinen enthält, die helfen, Informationen zwischen Elementen innerhalb des PCs, z.B. beim Start, zu übertragen. Auf der Festplatte, der Magnetplatte, der (magnetisch-) optischen Platte, dem ROM oder dem RAM können mehrere Programmmodule gespeichert werden, wie beispielsweise ein Betriebssystem, ein oder mehrere Anwendungsprogramme, wie das Verfahren zur Berechnung eines Sendebefehlssatzes und/oder andere Programmmodule und/oder Programmdaten. Ein Benutzer kann Befehle und Informationen in den Computer über Eingabevorrichtungen, wie beispielsweise eine Tastatur und ein Zeigegerät, eingeben. Andere Eingabevorrichtungen wie Mikrofon, Satellitenschüssel, Scanner oder dergleichen können ebenfalls enthalten sein. Diese und andere Eingabegeräte werden oft über eine serielle Schnittstelle, die mit dem Systembus gekoppelt ist, mit der Verarbeitungseinheit verbunden. Eingabegeräte können jedoch auch über andere Schnittstellen verbunden werden, wie z.B. über eine parallele Schnittstelle, oder einen universellen seriellen Bus (USB). Ein Monitor (z.B. eine GUI) oder eine andere Art von Anzeigevorrichtung kann auch über eine Schnittstelle, wie z.B. einen Videoadapter, an den Systembus angeschlossen werden. Zusätzlich zum Monitor kann der Computer auch andere periphere Ausgabegeräte wie beispielsweise Lautsprecher und Drucker beinhalten.

Die Recheneinheit wird in einer Netzwerkumgebung bzw. in einem "Netzwerk" betrieben, die logische Verbindungen zu einem oder mehreren entfernten Computern (Knoten) definiert. Der entfernte Computer kann ein anderer Personal-Computer, ein Server, ein Router, ein Netzwerk-PC, eine Peer-Vorrichtung oder ein anderer gemeinsamer Netzwerkknoten sein und kann viele oder alle der oben beschriebenen Elemente in Bezug auf den Personal-Computer beinhalten. Zu den logischen Verbindungen gehören ein Local Area Network (LAN) und ein Wide Area Network (WAN), ein Intranet und das Internet.

Unter eine, "Speicher" oder einer "Speichereinheit" oder "Speichermodul" und dergleichen kann im Zusammenhang mit der Erfindung beispielsweise ein flüchtiger Speicher in Form von Arbeitsspeicher (engl. Random-Access Memory, RAM) oder ein dauerhafter Speicher wie eine Festplatte oder ein Datenträger oder z. B. ein wechselbares Speichermodul verstanden werden.

Unter einem "Knoten" (Sendeknoten, Empfangsknoten) kann im Zusammenhang mit der Erfindung beispielsweise eine Recheneinheit (wie oben beschrieben), ein Prozessor und/oder eine Speichereinheit zum Speichern von Programmbefehlen verstanden werden. Beispielsweise ist der Prozessor speziell dazu eingerichtet, die Programmbefehle derart auszuführen, damit der Prozessor Funktionen ausführt, um das erfindungsgemäße Verfahren oder einen Schritt des erfindungsgemäßen Verfahrens zu implementieren oder realisieren. Die Knoten sind mit entsprechenden Netzwerkschnittstellen ausgebildet.

Unter "Bereitstellen", insbesondere in Bezug auf den Sendebe-fehls(daten)satz, kann im Zusammenhang mit der Er-findung beispielsweise ein rechnergestütztes Bereitstellen verstanden werden. Das Bereitstellen erfolgt beispielsweise über eine Schnittstelle (z. B. eine Datenbankschnittstelle, eine Netzwerkschnittstelle, eine Schnittstelle zu einer Speichereinheit). Über diese Schnittstelle können beim Bereitstellen entsprechende Daten und/oder Informationen z.B. an externe elektronische Instanzen übermittelt und/oder gesendet und/oder abgerufen und/oder empfangen werden. Dazu kann ein Kommunikationsmodul, wie z. B. eine Ethernetschnittstelle oder Netzwerkkarte, vorgesehen sein.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird dabei der Fachmann auch Einzelaspekte als Verbesserung oder Ergänzungen zu der jeweiligen Grundform der vorliegenden Erfindung hinzufügen.

Der Begriff "Sendebefehlsdatensatz" ist ein digitaler Datensatz, der mehrere Elemente umfassen kann. Er dient zur Steuerung des Sendevorgangs und umfasst im Kern den Sendebefehl für das Datenpaket. Darüber hinaus kann er den Sendevorgang hinsichtlich seiner technischen Details weiter spezifizieren. Welche technischen Parameter dabei Berücksichtigung finden sollen kann in einer bevorzugten Ausführungsform der Erfindung konfigurierbar sein. Vorzugsweise umfasst der Sendebefehlsdatensatz einen Sendezeitpunkt und die Form der Übertragung (monolithisch oder in zerlegten Komponenten).

Der Begriff "Übertragungserfordernis" meint die technischen Voraussetzungen, die zur Übertragung des Datenpaktes über das Netzwerk erfüllt sein müssen, so dass eine sichere und zuverlässige Übertragung sichergestellt werden kann. Dazu können z.B. insbesondere eine minimale Bandbreite, Anzahl von intermediären Knoten, Verwendung von Verschlüsselungstechnologie, maximal zulässige Zeitdauer der Übertragung und/oder ein Zeitpunkt oder eine Zeitphase der Übertragung zählen. Weitere Beispiel sind ein Hin- und Rückfluss der Daten, eine Dauer von Befehlsendung bis Ausführung und eventuelle Rückmeldung dazu.

Der Begriff "Netzwerk-Kenndaten" bezieht sich auf die technischen Eigenschaften und Merkmale des Netzwerkes. Diese sind typischerweise über die Zeit veränderlich. Dazu können insbesondere eine minimale und maximale Bandbreite, eine Betriebsweise des Netzwerkes und/oder eine maximale Auslastung zählen. Zu der Betriebsweise eines Netzwerkes zählen unter die Netzwerktopologie, wie z.B. ein ringförmiges Netzwerk oder ein lineares Netzwerk. Z.B. könnte in einem Kliniksystem ein ringförmiges Netzwerk bei Ausfall intelligent einen anderen Übertragungsweg auswählen. Das externe Versenden könnte z.B. in einem linearen Netzwerk bestimmter Architektur erfolgen. Des Weiteren können aus den Netzwerk-Kenndaten weitere Kenndaten abgeleitet werden. Es könnten z.B. Engpässe in einer 10Mbit/s- oder einer 100 Mbit/s-Leitung vorhanden sein oder die Performance einer Leitung kann sich verschlechtern, da ein Optokoppler schwach wird oder Kabelanschluss sich gelockert hat. Es kann aber auch sein, dass die Daten beispielsweise über ein Funknetzwerk/Sattelitennetz übertragen werden und dort weiteren Einflüssen ausgesetzt sind (Wetter, Zustand der Sendemasten etc.). Diese über die Zeit variablen Größen werden in ihrem aktuellen Zustand erfasst und zur Berechnung verwendet.

Das trainierte Modell ist ein Computermodell, das den Netzwerkverkehr mit seinen technischen Parametern, wie z.B. einer minimalen, maximalen und verfügbaren Bandbreite, modelliert. "Trainiert" meint in diesem Zusammenhang, dass das Netzwerk auf einen bestimmte Aufgabe hin auf Basis von Vorwissen, in Form von Trainingsdaten, trainiert worden ist, insbesondere auf die Berechnung von Befehlsdaten zum Auslösen der Datenübertragung eines Datenpaketes, so dass dessen Übertragungserfordernisse bei den jeweils herrschenden Netzwerkbedingungen oder bei dem jeweiligen Netzwerkzustand, der anhand der Netzwerk-Kenndaten ermittelt wird, eingehalten werden können. Die Trainingsdaten umfassen auch die realen Übertragungsbedingungen, insbesondere die reale Übertragungszeit. So kann eine Verknüpfung gelernt werden zwischen (real benötigter bzw. resultierender) Übertragungszeit für ein Datenpaket mit bestimmten Übertragungserfordernissen bei bestimmten Netzwerk-Kenndaten. Eine ausreichende Menge von Trainingsdaten erlaubt das Erzeugen eines Lernmodells. Anhand des Lernmodells ist es möglich, einen Sendebefehlssatz zu erzeugen, der insbesondere angibt, wann das Datenpaket unter den aktuellen Netzwerkbedingungen (Kenndaten) optimalerweise versendet werden sollte, damit die Übertragungserfordernisse sicher eingehalten werden können.

Das Modell ist ausgebildet, den Sendebefehlssatz so zu erzeugen, dass bei Übersendung des Datenpaktes die Netzwerkbelastung möglichst gering ist und/oder dass die erfassten Übertragungserfordernisse sicher eingehalten werden können (die z.B. auch eine minimale Netzwerkkapazität beinhalten können).Das Modell berücksichtigt zur Erzeugung des Sendebefehlssatzes den IST-Zustand des Netzwerkes mit vorkonfigurierbaren Parametern, wie Verlaufsdaten, Netzwerkauslastung, Netzwerkbelastungsspitzen etc. Das Modell ist ferner ausgelegt, auch Vorhersagen über die zukünftige Netzwerkbelastung bereitzustellen, basierend auf den gesammelten und erfassten Daten. Damit kann das Modell auch im Rahmen von Predictive Maintenance Maßnahmen zur Überwachung der Güte des Netzwerkes und/oder der daran angeschlossenen Knoten angewendet werden und Prognosedaten umfassen. Beispielsweise: Wenn aufgrund der Analyse von historischen Daten darauf geschlossen werden kann, dass demnächst (z.B. nachts) ein Netzwerkzustand erreicht sein wird, der eine höhere Bandbreite ermöglicht, so kann die Versendung der Datenpakte auf diesen Zeitraum hin geplant werden. Damit kann der technische Vorteil erreicht werden, dass auch bei Netzwerken mit einer geringeren Übertragungskapazität als von den Übertragungserfordernissen gefordert, eine zuverlässige und sichere Übertragung sichergestellt werden kann, indem das Modell einen solchen Sendebefehlssatz erzeugt, der definiert, dass das Datenpaket nur in Zeiten geringer Auslastung (und ausreichender Übertragungsrate) und/oder in zerlegter und/oder komprimierter Form, nämlich in kleineren Teilpakten zerlegt, ansteuert. Des Weiteren können damit Störungen im Vorfeld und vorab berechnet und z.B. im Rahmen eines Predictive Maintenance eingesetzt werden. Damit können indirekt physikalische Netzwerkfehler (z.B. Hardwareprobleme, Verbindungsabbruch) automatisch detektiert und gemeldet werden. Insgesamt ist eine intelligente Anpassung an Netzschwankungen (Verbesserungen und Verschlechterungen der Bandbreite) möglich.

Das Modell kann ein trainiertes neuronales Netzwerk oder ein anderes Modul der künstlichen Intelligenz (KI) sein. Das KI-Modul oder das Modell liefern in dieser Anwendung Stellgrößen, mit denen der Befehl zur Versendung des Datenpaktes so erzeugt bzw. eingestellt werden kann, dass ein vorgegebenes Übertragungserfordernis erzielt wird. KI-Module für solche Anwendungen werden zum großen Teil mit Erfahrungswissen aus historischen aufgezeichneten Datenübertragungen oder anderen empirischen Daten oder Simulationsdaten trainiert. Die Input-Neuronen umfassen somit das Datenpaket als solches, dessen Übertragungserfordernisse und die aktuellen Netzwerkbedingungen (Kenndaten). Die Output-Neuronen liefern den Sendebefehlssatz mit einem Sendezeitpunkt (oder -phase) und optional noch weiteren Sendeparametern. Die Architektur des Netzwerkes ist abhängig davon, wie detailliert der Sendebefehlssatz berechnet werden soll. In einer bevorzugten Ausführungsform der Erfindung wird ein rekurrentes Netz angewendet. Rekurrente Netze besitzen auch rückgerichtete (rekurrente) Kanten (feedback loops) und enthalten somit eine Rückkopplung. Solche Kanten werden dann häufig mit einer Zeitverzögerung versehen, sodass bei einer schrittweisen Verarbeitung die Neuronenausgaben der vergangenen Einheit wieder als Eingaben angelegt werden können. Diese Rückkopplungen ermöglichen einem Netz ein dynamisches Verhalten und statten es quasi mit einem Gedächtnis aus. Weiterhin kann ein Faltungsnetz (CNN, convolutional neural network) und/oder ein Netz mit zusätzlichen Zwischenschichten verwendet werden.

Die Erfassung der Netzwerk-Kenndaten kann mittels eines Datensammlers (Sniffer) erfolgen. Dabei ist es vorzugsweise in einer vorgeschalteten Konfigurationsphase konfigurierbar, welche Kenndaten erfasst werden sollen.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: eine schematische Darstellung eines Steuerknotens zur Steuerung des Sendevorganges für ein Datenpaket gemäß einer bevorzugten Ausführungsform der Erfindung;
- Fig. 2: eine weitere Darstellung eines Steuerknotens mit weiteren strukturellen Einheiten gemäß einer bevorzugten Ausführungsform der Erfindung;
- Fig. 3: eine schematische Darstellung eines modifizierten Datenpaketes;
- Fig. 4: eine weitere schematische Darstellung eines modifizierten Datenpaktes in einer alternativen Ausführungsform der Erfindung und
- Fig. 5: ein Ablaufdiagramm eines Verfahrens in einer bevorzugten Ausführungsform der Erfindung.

Im Folgenden werden weitere Ausführungsbeispiele im Zusammenhang mit den Figuren näher erläutert.

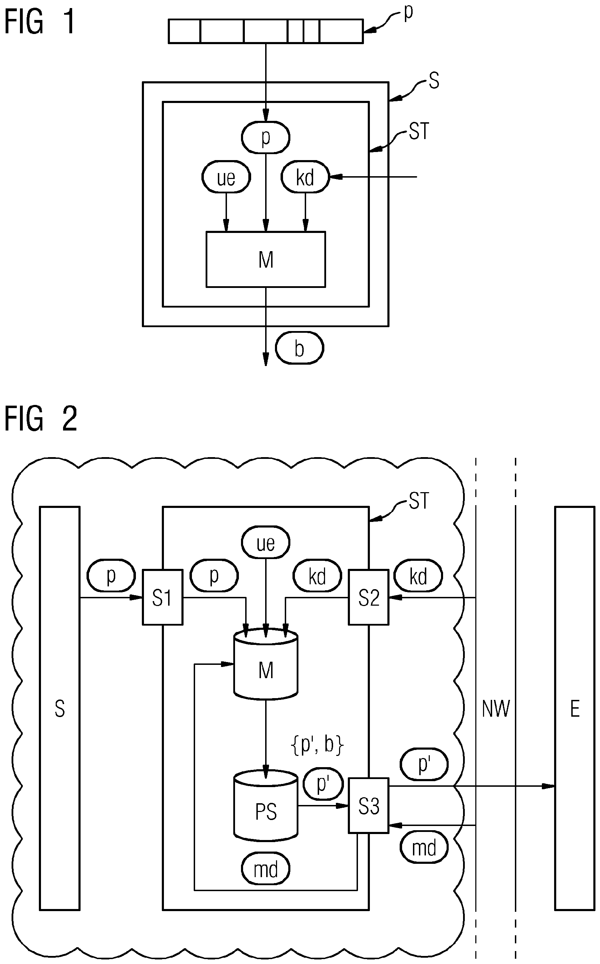

Fig. 1 zeigt in einer übersichtsartigen Schemadarstellung einen computer-implementierten Steuerknoten ST, der auf einem Sendeknoten S implementiert sein kann und den Datenverkehr zwischen dem Sendeknoten S und einem oder mehreren Empfangsknoten E analysiert, um die Datenübertragung zu steuern. Wie in Fig. 1 angedeutet, soll ein Datenpaket p oder eine Menge von Datenpaketen von dem Sendeknoten S an den zumindest einen Empfangsknoten E übermittelt werden. Dazu wird das Datenpaket p zunächst und vor der Übertragung an den Steuerknoten ST übermittelt. Da der Steuerknoten ST vorzugsweise in dem Sendeknoten angeordnet ist, ist hierzu kein Zugriff auf das Netzwerk erforderlich. Aus dem zu übertragenden Datenpaket p kann der Steuerknoten ST die Übertragungserfordernisse berechnen. Dazu kann er z.B. auf eine Regelbasis oder einen Speicher zugreifen. Alternativ ist es möglich, dass die Übertragungserfordernisse ue mit dem Datenpaket p übermittelt werden und der Steuerknoten ST diese nicht berechnen muss. Weiterhin werden Kenndaten kd von dem Netzwerk NW erfasst, über die das Datenpaket p an den Empfangsknoten E übertragen werden soll. Alle erfassten oder berechneten Daten p, ue, kd werden einem trainierten Modell M zugeführt, das dazu ausgebildet ist, in Antwort auf die zugeführten Werte einen Sendebefehlssatz b zu berechnen. Der Sendebefehlssatz b kann mehrere Felder umfassen und spezifiziert einen optimalen Zeitpunkt oder eine solche Zeitphase, die am besten geeignet ist, um das Datenpaket über das Netzwerk NW zu versenden, so dass dessen Übertragungserfordernisse ue eingehalten werden können.

Fig. 2 zeigt weitere Komponenten der beteiligten Knoten. Der Steuerknoten ist hier als separater Knoten ausgebildet, der allerdings über ein hoch-performantes Netzwerk mit dem Sendeknoten in Datenaustausch steht. Dies soll in Fig. 2 durch die geschwungene Umrandung der beiden Knoten S, St zum Ausdruck gebracht werden. Zum Erfassen des Datenpaketes p ist eine erste Schnittstelle S1 vorgesehen. Das Erfassen der Netzwerk-Kenndaten kd erfolgt über eine zweite Schnittstelle S2. Die Übertragungserfordernisse ue können dem Steuerknoten übermittelt werden, z.B. mit dem Datenpaket p ebenfalls über die erste Schnittstelle oder die Übertragungserfordernisse ue können lokal auf dem Steuerknoten ST berechnet werden, ggf. unter Zugriff auf einen Speicher und/oder mittels eines Algorithmus auf Basis des erfassten Datenpaktes p. Diese Daten p, ue, kd werden dem trainierten Modell M zugeleitet, das daraufhin den Sendebefehlssatz b erzeugt. Da der Sendebefehlssatz b möglicherweise erst eine Datenübertragung des Datenpaketes p zu einem späteren Zeitpunkt ansteuert, müssen die Daten bzw. das Datenpaket p zwischengespeichert werden. Dazu ist vorzugsweise in dem Steuerknoten ST und in dem Sendeknoten S ein Pufferspeicher PS angeordnet.

In einer bevorzugten Ausführungsform der Erfindung kann durch Anwendung des Modells M ein modifiziertes Datenpaket p' erzeugt werden. Das modifizierte Datenpaket p' kann im Vergleich zum (originalen) Datenpaket p erweitert sein. Insbesondere können ein Kennungsfeld fd und/oder ein Anforderungsfeld af ausgebildet sein.

Das Kennungsfeld kf dient zur Speicherung einer berechneten Priorität des Datenpaktes p. Diese kann numerisch oder in vorkonfigurierbaren Klassen repräsentiert sein und z.B. eine hohe Priorität definieren, wenn das Datenpaket p Totalausfalldaten repräsentiert und z.B. eine niedrige Priorität definieren, wenn das Datenpaket p eine Routinesendung betrifft, die ohnehin wiederholt wird. Durch die Erfassung dieses Parameters (Priorität) kann das Modell M Prioritätsmuster erkennen und für zukünftige Berechnungen nutzen.

Das Anforderungsfeld af dient zur Speicherung einer Aktivierungsfunktion, über die es einem externen Knoten, z.B. dem Empfangsknoten E, ermöglicht wird, das Datenpaket p spezifisch anzufordern.

In einem bevorzugten Ausführungsbeispiel wird das reale Ergebnis der Übertragung des Datenpaketes p, z.B. in Form der tatsächlich benötigten Übertragungszeit, gemessen. Es können auch weitere Messdaten md ermittelt werden, wie z.B. eine Güte der Übermittlung etc. Diese Messdaten md werden dann an den Steuerknoten ST zurückgespielt und können in einer Rücckopplungsschleife dem Modell M zugeführt werden. Dies ist schematisch in Fig. 2 durch den mit dem Bezugszeichen md gekennzeichneten Pfeil repräsentiert. Damit kann das Modell adaptiv und selbstlernend ausgebildet werden. So können z.B. insbesondere die Gewichtungen zwischen den Neuronen des Netzwerkes modifiziert werden, falls die realen Messdaten md zeigen, dass die geforderten Übertragungserfordernisse ue nicht in zufriedenstellendem Maße eingehalten werden konnten.

Das Modell kann trainiert worden sein mit Trainingsdaten und/oder mit Referenzdaten von anderen Sendeknoten-Netzwerk-Empfangskonten-Systemen und/oder mit historischen Daten.

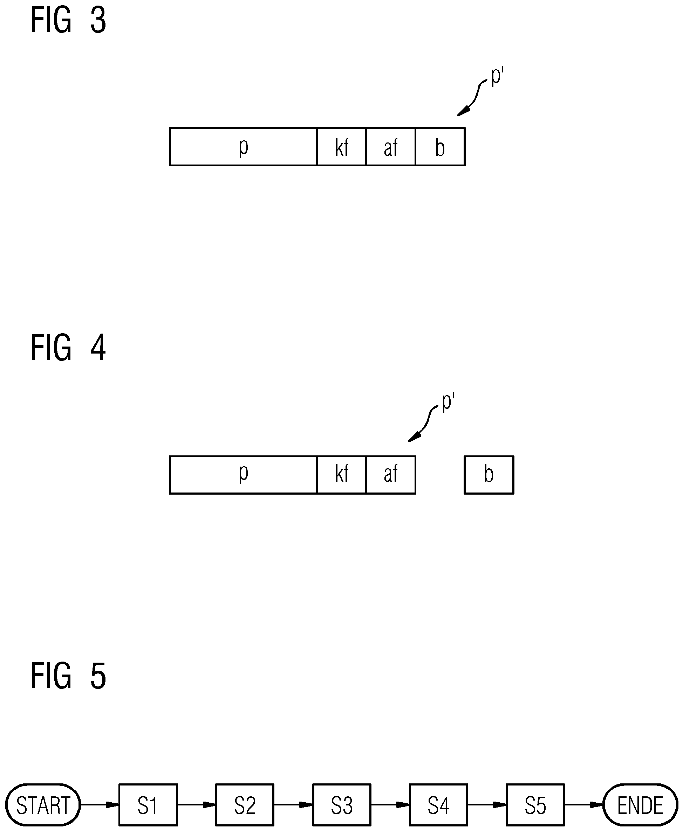

Dies ist in Fig. 3 schematisch dargestellt. Das modifizierte Datenpaket p' umfasst den berechneten Sendebefehlssatz b und kann neben den Datenpaket p noch das Kennungsfeld kf und das Anforderungsfeld af aufweisen. Alternativ ist es möglich, dass das originale Datenpaket p separat gespeichert wird.

In Fig. 4 ist eine alternative Ausführungsform gezeigt. In dieser ist an das originale Datenpaket p das Kennungsfeld kf und/oder das Anforderungsfeld af angehängt, während der Sendebefehlssatz b separat in einer Datenstruktur gespeichert ist, die der jeweils anderen Komponente, nämlich dem modifizierten Datenpaket p' zugeordnet ist.

Fig. 5 zeigt ein Ablaufdiagramm eines Steuerungsverfahrens gemäß einer bevorzugten Ausführungsform der Erfindung. Nach dem Start des Verfahrens erfolgt in Schritt S1 das Erfassen des zu übertragenden Datenpaketes p. In Schritt S2 werden die Übertragungserfordernisse ue für das jeweilige Datenpaket p ermittelt. Dies kann entweder durch eine Berechnung - z.B. unter Zugriff auf einen Speicher und unter Anwendung automatischen Berechnungsverfahrens - erfolgen oder die Übertragungserfordernisse ue werden direkt mit dem Datenpaket p erfasst. In Schritt S3 werden die aktuellen Kenndaten kd des Netzwerkes NW, über welches das Datenpaket p übermittelt werden soll, erfasst und können dann auf dem Steuerknoten ST analysiert werden. Alle bereitgestellten Größen p, ue, kd werden dann dem Modell M zugeführt, das in Schritt S4 den Sendebefehlssatz b für diese Daten berechnet und in Schritt S5 bereitstellt und dabei insbesondere einen Zeitpunkt für eine Übertragung festlegt. Zur Vorbereitung der Übertragung wird das Datenpaket p in dem Pufferspeicher PS zwischengespeichert. Daraufhin kann das Verfahren für das nächste Datenpaket p angewendet oder - z.B. im Fehlerfall - wiederholt werden, bevor das Verfahren endet.

Abschließend sei darauf hingewiesen, dass die Beschreibung der Erfindung und die Ausführungsbeispiele grundsätzlich nicht einschränkend in Hinblick auf eine bestimmte physikalisehe Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für medizinische Bilddaten, sondern auch für andere medizinische Daten angewendet werden kann. Ebenso kann es für Datenpakete angewendet werden, die in Hausnetzwerken (SmartHome) oder in Stromnetzen anfallen und über ein entsprechendes Netzwerk übertragen werden müssen. Des Weiteren können die Bauteile des Steuerknotens ST auf mehreren physikalischen Produkten verteilt realisiert sein.

## Patentansprüche

1. Computer-implementiertes Verfahren zum Erzeugen eines Sendebefehlssatzes (b) zur Steuerung der Übertragung von Datenpaketen (p) über ein digitales Netzwerk (NW), bei dem ein trainiertes Modells (M) bereitgestellt ist, das in einem Speicher gespeichert ist und das trainiert ist, um für ein jeweiliges Datenpaket (p) mit entsprechenden Übertragungserfordernissen (ue) unter Berücksichtigung von Netzwerk-Kenndaten (kd) einen Sendebefehlssatz (b) derart zu berechnen, dass die Übertragungserfordernisse (ue) bei Übertragung des Datenpaktes (p) erfüllt werden können, umfassend folgende Verfahrensschritte:
- Erfassen (S1) des zu übertragenden Datenpaketes (p);
- Berechnen (S2) von Übertragungserfordernissen (ue) des zu übertragenden Datenpaketes (p);
- Erfassen (S3) von aktuellen Netzwerk-Kenndaten (kd);
- Anwenden des trainierten Modells (M) mit den erfassten Übertragungserfordernissen (ue) und den erfassten aktuellen Netzwerk-Kenndaten (kd) zur Berechnung (S4) und Bereitstellung (S5) des Sendebefehlssatzes (b).

2. Verfahren nach Patentanspruch 1, wobei das trainierte Modell (M) zur Anwendung von Verfahren zum maschinellen Lernen dient und wobei Messdaten (md) und insbesondere die real gemessenen Übertragungslaufzeiten dem trainierten Modell (M) in einer Rückkopplungsschleife zurückgespeist werden.

3. Verfahren nach einem der vorangehenden Patentansprüche, bei dem das trainierte Modell (M) ein neuronales Netzwerk ist.

4. Verfahren nach einem der vorangehenden Patentansprüche, bei dem der Sendebefehlssatz (b) einen Zeitparameter und/oder einen Zerlegungsparameter umfasst, wobei der Zeitparameter definiert, zu welchem Zeitpunkt der Sendebefehl umgesetzt werden soll und wobei der Zerlegungsparameter definiert, ob und wenn ja, wie das zu übertragende Datenpaket (p) in Teilpakte zerlegt werden soll, die unabhängig und getrennt voneinander übertragen werden.

5. Verfahren nach einem der vorangehenden Patentansprüche, bei dem das trainierte Modell (M) mit Trainingsdaten und einem Verfahren zum überwachten Lernen trainiert worden ist.

6. Verfahren nach dem unmittelbar vorangehenden Patentanspruch, bei dem die Trainingsdaten Referenzdaten, Plandaten und/oder Simulationsdaten umfassen.

7. Verfahren nach einem der vorangehenden Patentansprüche, bei dem die zu übertragenden Datenpakete (p) nach Priorität kategorisiert werden, wobei die Priorität vorzugsweise vom Sendeknoten (S) automatisch und insbesondere durch Anwendung eines Analysealgorithmus auf den Inhalt des Datenpaketes (p) bestimmt wird.

8. Verfahren nach einem der vorangehenden Patentansprüche, bei dem alle oder ausgewählte zu übertragenden Datenpakete (p) mit einem Kennungsfeld (kf) erweitert werden, wobei das Kennungsfeld (kf) zumindest eine Angabe zur Priorität des Datenpaktes (p) umfasst.

9. Verfahren nach einem der vorangehenden Patentansprüche, bei dem alle oder ausgewählte zu übertragenden Datenpakete (p) mit einem Anforderungsfeld (af) erweitert werden, wobei das Anforderungsfeld (af) eine Aktivierungsfunktion umfasst, über die das jeweilige Datenpaket (p) von einem externen Empfängerknoten (E) angefordert werden kann.

10. Steuerknoten (ST) zur Steuerung der Übertragung von Datenpaketen (p) von einem Sendeknoten (S) an einen Empfangsknoten (E) über ein digitales Netzwerk (NW), wobei der Steuerknoten (ST) ausgebildet ist, ein Verfahren nach einem der vorangehenden Verfahrensansprüche auszuführen.

11. Steuerknoten (ST) nach dem vorangehenden Anspruch, der eine erste Schnittstelle (S1) umfasst zur Erfassung von Übertragungserfordernissen (ue) und eine zweite Schnittstelle (S2) zur Erfassung von aktuellen Netzwerk-Kenndaten (kd) sowie eine dritte Schnittstelle (S3) zur Ausgabe des Sendebefehlssatzes (b) und/oder zum Empfang von Messdaten (md) zu der Datenpaketübertragung.

12. Steuerknoten (ST) nach einem der vorangehenden auf den Steuerknoten (ST) bezogenen Ansprüche, der einen Modellspeicher umfasst oder auf einen Modellspeicher zugreifen kann, in dem das trainierte Modell (M) abgelegt ist.

13. Steuerknoten (ST) nach einem der vorangehenden auf den Steuerknoten (ST) bezogenen Ansprüche, der einen Pufferspeicher (PS) umfasst oder auf einen Pufferspeicher (PS) zugreifen kann, in dem das zu übertragenden Datenpaket (p, p') vor Versendung zwischengespeichert ist.

14. Computerprogrammprodukt mit Programmcode für das Ausführen eines Verfahrens nach einem der vorangehenden Verfahrensansprüche, wenn das Computerprogramm auf einem elektronischen Gerät oder einem Computer ausgeführt wird.

15. Computerprogramm mit Programmcode für das Ausführen eines Verfahrens nach einem der vorangehenden Verfahrensansprüche, wenn das Computerprogramm auf einem elektronischen Gerät oder einem Computer ausgeführt wird.
